## Europäisches Patentamt

## European Patent Office

## Office européen des brevets

(11) Veröffentlichungsnummer: **0 217 011**

**A1**

(12) # EUROPÄISCHE PATENTANMELDUNG

(21) Anmeldenummer: **86109178.3**

(22) Anmeldetag: **04.07.86**

(51) Int. Cl.⁴: **A 61 N 1/36**

(30) Priorität: **06.07.85 DE 3524232**

(43) Veröffentlichungstag der Anmeldung:
**08.04.87 Patentblatt 87/15**

(84) Benannte Vertragsstaaten:
**AT CH DE FR GB IT LI NL SE**

(71) Anmelder: **Rodler, Hans**
**Pehamweg 1-5**
**A-8053 Graz-Neuhart(AT)**

(72) Erfinder: **Rodler, Hans**
**Pehamweg 1-5**
**A-8053 Graz-Neuhart(AT)**

(54) **Universelles Elektrotherapiegerät.**

(57) Universelles Elektrotherapiegerät zur Erzeugung unterschiedlicher Reizströme, Interferenzströme oder Magnetfelder zur Behandlung von Patienten über Elektroden oder Magnetspulen. Hochfrequenz- oder Mittelfrequenzimpulse werden mit einer dem Reizstrom entsprechenden Spannungsgröße pulspausen- oder pulsbreitenmoduliert und diese modulierten Impulse mittels elektronischen Leistungsschaltern (3) verstärkt und diese über Tiefpaß- oder Bandpaßfilter (4) dem Patienten zugeführt. Eine Intensitätsregeleinrichtung (6,7,8,9), die auf die Höhe der Modulationsspannung einwirkt, bestimmt hiebei die Stromstärke im Patientenkreis.

Fig. 1

EP 0 217 011 A1

Universelles Elektrotherapiegeraet

Die Erfindung betrifft ein Elektrotherapiegeraet entsprechend dem Oberbegriff des Anspruchs 1.

Bei Elektrotherapiegeraeten wird ein Strom verschiedener Kurvenform oder magnetische Impulse dem menschlichen Krper zugefuehrt. Es sind hierzu bestimmte Stromstaerken erforderlich, die in Leistungsverstaerkern verstaerkt werden. Die bisher bekannten Anordnungen arbeiten mit analogen Endverstaerkern, wobei ein Funktionsgenerator die entsprechende Kurvenform erzeugt. Der Strom wird ueber Leistungsverstaerker verstaerkt und dem Patienten direkt zugefuehrt. Bei dieser Form der Behandlung besteht die Gefahr, dass bei Kurzschluss des Leistungstransistors der Patient hohen Stromstaerken ausgesetzt werden kann. Ausserdem ist besonders bei Konstantstromgeraeten der Wirkungsgrad sehr unguenstig, da eine hohe Spannung vorhanden sein muss, sodass im Leistungstransistor bei grossen Stroemen eine hohe Verlustleistung auftritt.

Aufgabe der Erfindung ist es, ein Geraet zu schaffen, das eine sichere, universellere und bessere Elektrotherapie ermoeglicht. Die Aufgabe wird entsprechend dem Anspruch 1 geloest.
Das Wirkungsprinzip ist hierbei folgendermassen:
Schmale Impulse mit hoher Frequenz werden mit der gewuenschten Kurvenform pulspausen- und pulsbreitenmoduliert und durch elektronische Leistungsschalter verstaerkt. Diese Impulse werden einem Tiefpass- oder Bandpassfilter zugefuehrt und fuehren von diesem direkt zum Patienten. Das Filter bewirkt, da es sich um Gleichstromimpulse handelt, dass aus den Hochfrequenzimpulsen ein kontinuierlich fliessender Gleichstrom der gewuenschten Kurvenform entsteht. Durch entsprechendes Format der Impulse koennen auch Wechselstroeme auf diese Art und Weise erzeugt werden. Die entstehende Kurvenform des Ausgangsstromes wird durch den Abstand und (oder) durch die Breite der Hochfrequenzimpulse bestimmt. Ebenso ist die Hoehe des Ausgangsstromes durch den Impulsabstand und (oder) durch die Impulsbreite gegeben. Diese schmalen Impulse koennen transformiert werden, sodass mit kleinen Batteriespannungen hohe Ausgangsspannungen erzielt werden. Fuer sehr hohe Frequenzen kann auch der menschliche Koerper als Tiefpassfilter betrachet werden, sodass fuer verschiedene Anwendungsfaelle diese Impulse direkt dem Koerper zugefuehrt werden. Es koennen auch beide Modulationsformen - Pulspausenmodulation und Pulsbreitenmodulation - gleichzeitig angewendet werden, wobei die eine z. B. zur Modulation der Kurvenform und die andere zur Intensitaetsregelung herangezogen werden kann. Da Tiefpassfilter sehr klein aufgebaut werden koennen und die Uebertragung mittels Hochfrequenztransformator moeglich ist, koennen diese Tiefpassfilter zusammen mit den Elektroden auch inplantiert werden und von der Koerperoberflaeche aus durch die Primaerspule des Transformators die Impulse zugefuehrt werden. Bei Verwendung von Transformatoren muessen an der Sekundaerseite die Hochfrequenzimpulse gleichgerichtet werden. Da auf diese Weise auch Wechselstroeme erzeugt werden koennen, koennen mittels zwei oder mehreren Kanaelen Interferenzstroeme sowohl nach dem Frequenzunterschiedsprinzip als auch nach dem Phasenmodulationsprinzip gebildet werden. Es ist daher moeglich, ein Geraet zu schaffen, das sowohl Gleichstroeme als auch Gleichstromimpulse verschiedenster Kurvenform, Hochfrequenzimpulse, Wechselstroeme oder Interferenzstroeme produziert. Durch entsprechende Wandler koennen daher mit demselben Geraet ausserdem Ultraschall- oder mittels Magnetspulen auch Magnetfeldbehandlungen durchgefuehrt werden. Dadurch, dass anstelle von analogen Leistungsverstaerkern elektronische Leistungsschalter Verwendung finden, die mittels einer Pulspausen- oder Pulsbreitenmodulation betrieben werden, tritt an den Endstufen nur ein geringer Leistungsverlust auf. Ausserdem kann die Uebertragung zum Patienten mittels Transformatoren erdungsfrei erfolgen, sodass bei Kurzschluss eines elektronischen Leistungsschalters der Patient nicht belastet wird. Durch das Bandpassfilter unmittelbar am Patientenanschluss wird erzielt, dass die Hochfrequenz ausgesiebt wird und nur die Huellkurve den Patienten erreicht. Durch Verwendung entsprechend hoher Frequenzen im Bereich von ueber 10 KHz ist gewaehrleistet, dass auch Kurvenformen mit grossen Anstiegsgradianten erzeugt werden. Da die Konstantstromregelung ausserhalb der Leistungsverstaerkung stattfindet, ergibt sich auch bei Konstantstromgeraeten ein guter Wirkungsgrad, sodass derartige Geraete vorteilhaft auch mit Batteriebetrieb aufgebaut werden koennen.

Die Erfindung ist an einem Ausfuehrungsbeispiel naeher erlaeutert, und zwar zeigt
Fig. 1 das Prinzipschaltbild einer Puls-Pausenmodulation,
Fig. 2 das Prinzipschaltbild einer Pulspausen- und Pulsbreitenmodulation und
Fig. 3 eine Zweikanalausfuehrung fuer Interferenzstroeme.
Fig. 4 das Grundprinzip
Fig. 5 den Leistungschalter
Fig. 6 eine implatierte Anordnung.

Fig. 1
Der Hochfrequenz-Impulsgenerator 1 erzeugt Hochfrequenzimpulse mit einer Frequenz von mehr als 10 KHz.

Diese Hochfrequenzimpulse werden dem digitalen oder analogen Puls-Pausenmodulator 2 zugefuehrt. Dieser moduliert die Puls-Pausen, sodass Impulse mit unterschiedlichen Abstaenden je nach Modulationsspannung am Ausgang auftreten. Diese Impulse steuern den elektronischen Leistungsschalter 3, der mit jedem auftretenden Impuls auf die Dauer des Impulses durchschaltet und den hierdurch verstaerkten Impuls an das Filter 4, das als Bandpass- oder als Tiefpassfilter aufgebaut sein kann, abgibt. Da es sich hierbei um Gleichstromimpulse handelt, entsteht am Ausgang des Filters eine Huellkurve, die der aufgedrueckten Modulationsspannung entspricht. In dem angefuehrten Beispiel wird als Modulationsspannung ein Rechteckimpuls 20 verwendet, der in der Modulationsstufe in eine Impulsgruppe 21 umgewandelt wird. Am Ausgang des Filters entsteht wieder die Modulationsspannung 20, jedoch in verstaerkter Form. Die Filter koennen z. B. aus Induktivitaeten und Kapazitaeten aufgebaut werden. Die Hochfrequenz wird durch diese Filter 4 ausgesiebt, sodass am Ausgang eine Gleichspannungshuellkurve entsteht. Diese Gleichspannung entspricht der Form nach der aufgedrueckten Modulationsspannung. Um die Intensitaet dieser Spannung am Ausgang aendern zu koennen, wird die vom Funktionsgenerator 5 gelieferte Modulationsspannung 20 einem multiplizierenden Digital-Analogwandler 6 zugefuehrt, der von einem Zaehler 7 gesteuert wird, sodass am Ausgang entsprechend der Einstellung des Zaehlers 7 eine bestimmte Groesse der Modulationsspannung auftritt. Damit am Ausgang eine Konstantstromabhaengigkeit besteht, ist in Serie zum Patientenkreis 11 ein Widerstand 10 vorgesehen, von dem eine stromabhaengige Spannung abgegriffen wird und dem einen Eingang eines Differenzverstaerkers 9 zugefuehrt wird. Der zweite Eingang dieses Differenzverstaerkers 9 ist mit dem Ausgang des Digital-Analogwandlers 6 verbunden. Da die Verstaerkung derartiger Differenzverstaerker 9 die Eigenschaft haben, die Differenz zwischen den beiden Eingangsspannungen moeglichst gering zu halten, tritt am Ausgang eine Modulationsspannung auf, die bewirkt, dass im Patientenstromkreis 11 ein Strom fliesst, der in einem festen Verhaeltnis zur eingestellten Modulationsspannung steht. Durch diese Regeleinrichtung ist es moeglich, den Zaehler 7 mit einem Anzeigedisplay 12 zu verbinden, das in Stromwerten des Ausgangsstromes geeicht ist. Zur Steuerung des Zaehlers ist ein Impulsgenerator 8 vorgesehen, der ueber die Tasten 13 den Zaehler zum Vor- oder Rueckwaertszaehlen veranlasst, sodass mit diesen beiden Tasten jede beliebige Stromstaerke eingestellt werden kann. Die Konstantstromregelung kann ueber einen Schalter 14 abgeschaltet werden, da fuer bestimmte Behandlungen ein Konstantstrom unerwuenscht ist. Dieselbe Anordnung ist auch fuer Konstantspannungsausgaenge realisierbar, wobei in diesem Fall eine von der Ausgangsspannung abhaengige Spannung zum Verstaerker 9 rueckgefuehrt wird. Die ganze Anordnung kann mit einem Mikroprozessor aufgebaut werden, wobei die Modulationsfunktionen und die Stromformen in Speicher abgelegt werden.

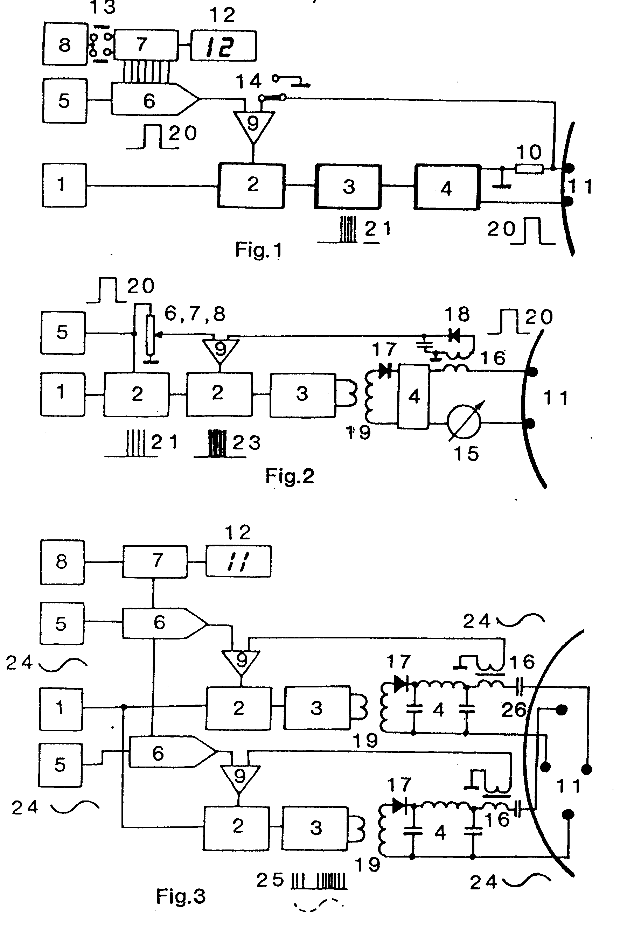

Fig. 2
Die vom Hochfrequenz-Impulsgenerator 1 gelieferten Impulse werden zunaechst im Puls-Pausenmodulator 2 entsprechend der vom Funktionsgenerator 5 gelieferten Modulationsspannung 20 puls-pausenmoduliert und anschliessend im zweiten Modulator 2, der als Pulsbreitenmodulator dient, von einer von der Intensitaetsregelung abhaengigen Modulationsspannung und vom Ausgangsstrom oder von der Ausgangsspannung abhaengigen Vergleichsspannung durch den Differenzverstaerker 9 pulsbreitenmoduliert. Durch diese doppelte Modulation ist eine bessere Aufloesung der Intensitaet und ein groesserer Modulationsgrad gewaehrleistet. Die elektronischen Leistungsschalter 3 verstaerken die Impulse und fuehren sie einem Hochfrequenzausgangstransformator 19 zu, der sekundaerseitig ueber den Gleichrichter 17 die hochtransformierten Impulse dem Filter 4 die Huellkurve entsprechend der Modulationsspannung entsteht. Der Impulstransformator 16 gewinnt aus der Restwelligkeit eine vom Patienten abhaengige Impulsspannung, die gleichgerichtet, gesiebt und dem Differenzverstaerker 9 zugefuehrt wird. Die zweite Seite des Differenzverstaerkers ist mit einer Potentiometereinrichtung 6,7,8 , die aehnlich der Abbildung 1, oder auch durch ein Potentiometer, gebildet sein kann, als Intensitaetsregler mit dem Funktionsgenerator 5 verbunden. 15 ist eine Stromanzeigeeinrichtung und 11 ist der Patientenstromkreis oder der zu behandelnde Koerper.

Fig. 3
zeigt eine Zweikanalausfuehrung zur Interferenzstrombehandlung. 1 ist der Hochfrequenzimpulsgenerator, der den Puls-Pausengeneratoren 2 Hochfrequenzimpulse zufuehrt, die in diesen puls-pausenmoduliert werden und durch die elektronischen Leistungsschalter 3 verstaerkt werden. Die Hochfrequenztransformatoren 16 sind sekundaerseitig mit dem Gleichrichter 17 verbunden, der die gleichgerichteten Hochfrequenzimpulse dem Filter 4 zufuehrt und ueber Kondensatoren die entstehende Wechselstromhuellkurve zum Patientenstromkreis 11 fuehrt. In Serie zu diesem Stromkreis liegt der Transformator 16, der, da es sich um Wechselstrom handelt, eine dem durchfliessenden Wechselstrom proportionale Spannung zu dem Differenzverstaerker 9 zurueckfuehrt, der anderseits von der

Modulationswechselspannung 24 ueber den Digital-Analogwandler 6 gesteuert wird. Zwischen den beiden Funktionsgeneratoren, die die Modulationsspannung fuer den Puls-Pausenmodulator erzeugen, kann entweder ein geringer Frequenzunterschied zur Erzeugung der Interferenz oder eine gesteuerte Phasenverschiebung bestehen. Bei der Frequenzunterschiedsmethode ist der eine Funktionsgenerator 5 ca. 4000 Hz, waehrend der andere zwischen 4001 und 4100 Hz variiert, sodass als Interferenzspannung im Patienten zwischen 1 und 100 Hz auftritt. Eine andere Methode benuetzt die Phasenmodulation. In diesem Falle sind die Frequenzen beider Funktionsgeneratoren gleich gross. Im einen Funktionsgenerator 8 wird die Phasenlage gegenueber dem anderen Funktionsgenerator 5 in der geforderten Interferenzkurvenform analog oder digital veraendert. Die Groesse der Modulationsspannung wird durch den multiplizierenden Digital-Analogwandler 6 und den Zaehler 7 gemeinsam fuer beide Kanaele gesteuert, wobei eine Anzeigeeinrichtung 12 am Zaehler 7 die Groesse des Stromes anzeigt. Durch die gemeinsame Steuerung mit einem Zaehler 7 ist gewaehrleistet, dass beide Stromstaerken gleich gross sind. 24 ist die Modulationsfrequenz von 4 KHz und 25 das Impulsbild der modulierten Sinuswelle. Am Ausgang tritt wieder das Modulationssignal 24 auf, das in der Tiefe des Behandlungsobjektes durch Interferenz die Interferenzkurve bildet. Durch die Kondensatoren 26 werden Gleichstromanteile ausgeschaltet, sodass als Huellkurve ein Wechselstrom entsteht.

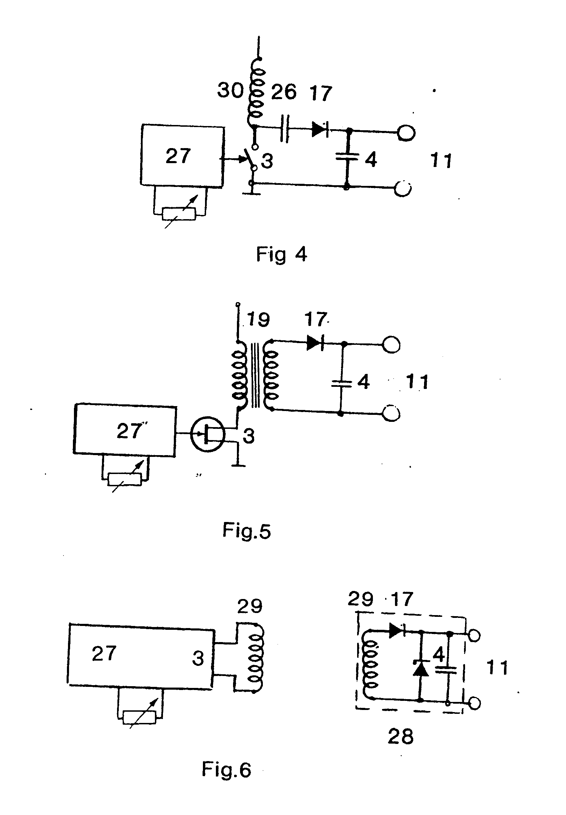

Fig.4 Das Grundprinzip besteht aus einem elektronischen Leistungschalter 3 der von der Modulations-und Steuereinrichtung 27 entsprechend der Pulsbreite eingeschaltet und entsprechend der Pausenbreite ausgeschaltet wird. Durch die Induktivitaet 30 als Arbeitswiderstand entsteht eine Rechteckspannung deren Amplitude nur von der angeschlossenen Spannungsversorgung abhaengt. Am Kondensator 26 der gleichzeitig zur Potentialtrennung dient , ensteht daher eine Rechteckspannung mit unterschiedlichen Pulsabstaenden oder -breiten ,sodass durch den Gleichrichter 17 ein Gleichstromimpulszug mit unterschiedlichen Energieinhalt entsteht. Durch das Tiefpassfilter 4 werden die Impulse von dem Patientenkreis 11 ferngehalten ,so das im Patientenkreis 11 nur die Hllkurve entsprechend dem unterschiedlichen Energieinhalt zur Wirkung kommt. Die Impulse knnen in ihrer Amplitude stabilisiert werden da die Patientenstromstaerke nur von der Pulsmodulation abhaengt. Aus diesem Prinzipschaltbild sieht man, dass weder ein Kurzschluss noch eine Unterbrechung im Leistungschalterkreis zu unzulaessigen Stromstaerken im Patientenkreis fuehrt.

Fig.5 In diesem Falle wird ein Leistungsfet als Schalter 3 benuetzt .27 ist die Steuer und -Modulationselektronik.Da die Impulse durch den Transformator 19 in ihrer Spannung erhoeht werden ,koennen als Versorgungsspannung niedrige Spannungen aus Batterien Verwendung finden .

Fig.6 zeigt eine Anordnung bei der nach der Modulations - Steuer und- Verstrkerstufe 27,3 eine induktive Antenne 29 vorgesehen ist ,die die Energie an die implatierte Antenne 29 abstrahlt.Der implatierte Teil 28 bentigt hiebei ausschliesslich passive Bauelemente und besteht aus einer Spule 29 als Antenne ,einer Diode 17 , einer Amplitudenstabilisierung und dem Tiefpassfilter 4 .In diesem Falle ist es zweckmssig Frequenzen mit mehr als 100 khz zu verwenden ,um gute Uebertragungseigenschaften zu erreichen. Die Stromstaerke und Spannung im Patientenkreis 11 ist auch bei dieser Anordnung nur von der Modulationsspannung im Steuergeraet 27 abhaengig .
Durch Verwendung eines Mikroprozessors fuer diese Anordnungen koennen verschiedene Kurvenformen in den Speichern abgelegt werden und dadurch universell anwendbare Therapiegeraete geschaffen werden.
Die elektronischen Leistungsschalter 3 zur . Leistungsverstaerkung geben einen wesentlich besseren Wirkungsgrad als analoge Leistungsverstaerker, da sie praktisch verlustfrei arbeiten, da ihr Zustand entweder durchgeschaltet oder offen ist. Besonders Konstantstromgeraete koennen hierdurch mit Batteriebetrieb aufgebaut werden, da ein hoher Wirkungsgrad erreicht wird.
Als elektronische Leistungsschalter koennen Leistungsfeldeffekttransistoren, PNP-, NPN-Transistoren, Triacs oder andere Halbleiterelemente eingesetzt werden.

Patentansprueche:

1.    Elektrotherapiegeraet    zur    Erzeugung    von
unterschiedlichen  Reizstroemen,  Interferenzstroemen  oder
Magnetfeldern  zum  Anschluss  an  einen  Patienten  ueber
Elektroden,  Transformatoren  und  Elektroden,  Magnetspulen,
Antennen      und      inplantierten      Antennen      oder
Ultraschallwandlern  und  Elektroden,  dadurch  gekennzeichnet,
dass
a)  Hochfrequenz-  oder  Mittelfrequenzimpulse  mit  einer  dem
Reizstrom  entsprechenden  Spannungsgroesse  analog  und/oder
digital  pulspausen-  und/oder  pulsbreitenmoduliert  werden,
die
b)   mit   elektronischen  Leistungsschaltern   (3)   verstaerkt
werden und
c)  ueber  Bandpass-  oder  Tiefpassfilter  (4)   dem  Patienten
zugefuehrt werden, wobei
d)   im  Patientenstromkreis  (11)  die  Huellkurve  zur  Wirkung
kommt und
e)    eine    dieser    Huellkurve    entsprechende    Strom-  oder
Spannungsgroesse   zu   einer    Intensitaetsregeleinrichtung
rueckgefuehrt wird, wobei
f)  die  Intensitaetsregeleinrichtung  gleichzeitig  durch
Zusammenwirken  mit  einer  Intensitaetseinstelleinrichtung
die Sollgroesse des Patientenstromes bestimmt.

2.  Elektrotherapiegeraet  entsprechend  dem  Oberbegriff  des
Anspruches 1, dadurch gekennzeichnet, dass
a)  ein Hochfrequenz- oder Mittelfrequenzgenerator (1) ueber
eine
b)     analoge     und/oder     digitale     Pulspausen- und/oder
Pulsbreitenmodulationseinrichtung (2)
c)  mit elektronischen Leistungsschaltern (3) verbunden ist,
deren Ausgang
d)  ueber  Bandpass- und/oder  Tiefpassfilter  (4)  mit  dem
Patientenstromkreis (11) in Verbindung steht, wobei
e) eine zum Patientenstromkreis (11) in Serie oder parallel
angeordnete   Vergleichseinrichtung   (10,16)   mit   einer
Intensitaetsregeleinrichtung   (6,7,8,9)   einerseits   und
anderseits
f)   mit   einem   Funktionsgenerator   (5)   ueber   eine
Intensitaetseinstelleinrichtung (7,8,13) verbunden ist und
der  Ausgang dieser Intensitaetsregeleinrichtung (6,7,8,9)
mit   dem   Modulationseingang   der   Pulsbreiten- oder
Pulspausenmodulationseinrichtung (2) verbunden ist.

3. Elektrotherapiegeraet entsprechend dem Oberbegriff des Anspruches 1, dadurch gekennzeichnet, dass zumindest zwei Modulationseinrichtungen (2) zumindest zwei elektronische Leistungsschalter (3), zwei Filtereinrichtungen (4) und zwei Funktionsgeneratoren (5,8) vorgesehen sind, wobei die zwei Funktionsgeneratoren (5,8) unterschiedliche Frequenzen oder Funktionen aufweisen oder zumindest einer eine Phasenmodulationseinrichtung besitzt.

4. Elektrotherapiegeraet nach Anspruch 1 - 2, dadurch gekennzeichnet, dass
a) eine zweite Modulationseinrichtung (2) vorgesehen ist, sodass sowohl eine Pulsbreitenmodulation als auch Pulspausenmodulation die HF- oder MF-Impulse moduliert, wobei zumindest eine Modulationseinrichtung (2) von der Intensitaetsregeleinrichtung (6,7,8,9) beeinflusst wird.

5. Elektrotherapiegeraet nach Anspruch 1, dadurch gekennzeichnet, dass nach den elektronischen Leistungsschaltern eine Sendeantenne und eine im Patienten inplantierte Empfangsantenne vorgesehen ist, an die eine Gleichrichter- und Amplitudenstabilisierung, sowie ein Tiefpassfilter angeschlossen ist, dessen Ausgang direkt mit den Nervenfasern verbunden ist.

6. Elektrotherapiegeraet nach Anspruch 1, dadurch gekennzeichnet, dass die Intensitaetsregeleinrichtung
a) aus einem multiplizierenden Analog-Digitalwandler (6) besteht, der einerseits mit einer digitalen Vor-Rueckwaertszaehleinrichtung (7), welche mittels Tasten oder Zeitschalter mit einem Impulsgenerator (1) und der anderseits
b) mit einem Funktionsgenerator (5) verbunden ist, wobei
c) der Ausgang des multiplizierenden Analog-Digitalwandlers mit einem Eingang eines Differenzverstaerkers, dessen zweiter Eingang mit der im Patientenkreis liegenden Vergleichseinrichtung (10,16) verbunden ist und der Ausgang des Verstaerkers am Modulationseingang des Pulspausen- oder Pulsbreitenmodulators angeschlossen ist.

7. Elektrotherapiegeraet nach Anspruch 1 - 3, dadurch gekennzeichnet, dass die Pulspausen-, Pulsbreiten-Modulationseinrichtung (2) aus einer digitalen Einrichtung besteht, die Impulse entsprechend der Modulationsspannung weglaesst oder hinzufuegt.

8. Elektrotherapiegeraet nach Anspruch 1 - 7, dadurch gekennzeichnet, dass die Modulationseinrichtung (2), der Funktionsgenerator (5) und die Regeleinrichtung (6,7,8,9) aus einem Mikrocomputer bestehen und die Stromformen und die Modulationsfunktionen in Speicher abgelegt sind.

9. Elektrotherapiegeraet nach Anspruch 1 - 4, dadurch gekennzeichnet, dass nach den Leistungsschaltern Transformatoren zum Hochtransformieren vorgesehen sind und die Sekundaerwicklung ueber Gleichrichter mit dem Band- oder Tiefpassfilter verbunden ist.

10. Elektrotherapiegeraet nach Anspruch 1 - 6, dadurch gekennzeichnet, dass die elektronischen Leistungsschalter aus Leistungsfeldeffekttransistoren, aus NPN-, PNP- Transistoren, Triacs oder anderen Halbleiterschaltelementen bestehen.

1/2

0217011

Fig. 1

Fig.2

Fig.3

Fig 4

Fig.5

Fig.6

# EUROPÄISCHER RECHERCHENBERICHT

| | EINSCHLÄGIGE DOKUMENTE | | EP 86109178.3 | |
|---|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int Cl 4)** | |
| A | AT - B - 345 970 (NEMEC)<br><br>* Patentansprüche 1,2 * | 1-3 | A 61 N 1/36 | |
| A | AT - B - 312 793 (INSTITUTUL)<br><br>* Seite 2, Zeile 52 - Seite 3, Zeile 5; Fig. 1 * | 1,2,5 | | |
| A | EP - A2 - 0 026 479 (SIEMENS)<br><br>* Zusammenfassung; Seite 4, Zeile 24 - Seite 5, Zeilen 6, 12-17; Fig. 1 * | 1,2 | | |
| A | US - A - 4 374 524 (HUDEK)<br><br>* Zusammenfassung; Fig. 1 * | 1-3,7 | | |
| A | US - A - 4 340 063 (MAURER)<br><br>* Zusammenfassung; Spalte 6, Anspruch 1; Fig. 2 * | 1,2 | **RECHERCHIERTE SACHGEBIETE (Int. Cl 4)**<br><br>A 61 N | |
| A | DE - A1 - 3 212 706 (EMPI)<br><br>* Zusammenfassung; Ansprüche 1-3; Fig. 3 * | 1,2 | | |
| A | DE - A1 - 3 207 050 (SIEMENS)<br><br>* Zusammenfassung; Ansprüche 1-4 * | 1,2,7, 8 | | |
| A | AU - B - 63 647/80 (LAMERS)<br><br>* Ansprüche 1,3; Fig. * | 1,2 | | |

Der vorliegende Recherchenbericht wurde für alle Patentansprüche erstellt

| Recherchenort | Abschlußdatum der Recherche | Prüfer |
|---|---|---|
| WIEN | 02-12-1986 | NEGWER |

**KATEGORIE DER GENANNTEN DOKUMENTEN**
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82